(19) Europäisches Patentamt

European Patent Office

Office européen des brevets

(11) EP 0 713 505 B1

(12) FASCICULE DE BREVET EUROPEEN

(45) Date de publication et mention
de la délivrance du brevet:
07.11.2001 Bulletin 2001/45

(51) Int Cl.⁷: C08G 69/10

(21) Numéro de dépôt: 94924342.2

(22) Date de dépôt: 09.08.1994

(86) Numéro de dépôt international:
PCT/FR94/00992

(87) Numéro de publication internationale:
WO 95/04772 (16.02.1995 Gazette 1995/08)

(54) **PROCEDE DE PREPARATION DE POLYAMINOACIDES**

VERFAHREN ZUR HERSTELLUNG VON POLYAMINOSÄUREN

METHOD FOR THE PREPARATION OF POLYAMINOACIDS

(84) Etats contractants désignés:
AT BE CH DE DK ES FR GB GR IE IT LI LU MC NL
PT SE

(30) Priorité: 10.08.1993 FR 9309991

(43) Date de publication de la demande:
29.05.1996 Bulletin 1996/22

(73) Titulaire: FLAMEL TECHNOLOGIES
69693 Venissieux Cédex (FR)

(72) Inventeurs:
• SOULA, Gérard
F-69330 Meyzieu (FR)
• GROSSELIN, Jean-Michel
F-69110 Sainte-Foy-lès-Lyon (FR)
• JORDA, Rafaël
F-69110 Sainte-Foy-lès-Lyon (FR)
• CASTAN, Catherine
F-69530 Brignais (FR)

(74) Mandataire: Fleurance, Raphael et al
Cabinet Plasseraud
27, rue de la Villette
69003 Lyon (FR)

(56) Documents cités:
GB-A- 996 760          GB-A- 1 024 393
GB-A- 1 202 765        US-A- 3 536 672

• H.R. KIRCHELDORF " Alpha-amino
acid-N-carboxyanhydrides and related
heterocycles"; Springer Verlag (1987), page 92.

EP 0 713 505 B1

**Description**

**DOMAINE TECHNIQUE :**

La présente invention appartient au domaine de la synthèse de polyaminoacides à partir de N-carboxyanhydrides d'aminoacides (ci-après dénommés NCA).

Plus précisément encore, la présente invention concerne la polymérisation, en milieu liquide, des NCA par des initiateurs de polymérisation de type base forte.

**TECHNIQUE ANTERIEURE :**

Les polyaminoacides sont des polymères biocompatibles et biodégradables qui trouvent des applications dans le domaine biomédical. En particulier, ils peuvent constituer des biomatériaux, par exemple utiles pour servir de matières premières pour la fabrication de prothèses, d'implants, ou bien encore pour être mis en oeuvre comme supports permettant la libération de principes actifs. Des applications des polyaminoacides à titre de fils de suture résorbables, de substituts cutanés temporaires ou de fibres textiles ont également été décrites.

Pour pouvoir être exploitables dans ces diverses applications, il importe que les polyaminoacides puissent se présenter sous une forme liquide ou semi-liquide, manipulable dans le cadre d'opérations de formage d'articles finis, tels que la filmature, la filature, le moulage ... Cela sous-tend donc une maîtrise de la viscosité intrinsèque et/ou de la masse molaire en poids $M_w$ du polyaminoacide synthétisé.

En plus de cela, et de façon antagoniste, il est souhaitable que ces polyaminoacides possèdent une masse molaire $M_w$ supérieure à un seuil que l'on peut fixer, par exemple, à $M_w = 50\ 000$, en deçà duquel ils ne satisfont pas aux caractéristiques structurelles et mécaniques, attendues dans les applications envisagées.

L'ouvrage de H. R. KRICHELDORF, "$\alpha$-aminoacid-N-carboxyanhydrides and related heterocycles", SPRINGER. VERLAG (1987), enseigne que la polymérisation des NCA est généralement effectuée en milieu liquide, à l'aide d'agents d'amorçage de polymérisation (dénommés également initiateurs) qui peuvent être des nucléophiles protiques, tels que l'eau, les amines primaires et les amines secondaires, ou des bases fortes, telles que les amines ou les phosphines tertiaires ou les alcoolates. Outre ces bases fortes, le brevet anglais GB-996 760 propose des organométalliques, tels que des trialkyl-lithiums ou aluminiums en tant qu'initiateurs de polymérisation de NCA.

KRICHELDORF précise, (page 92), que la polymérisation des NCA, initiée par des nucléophiles protiques, ne donne pas lieu à des hauts poids moléculaires (DPn inférieur à 200), à la différence des initiateurs du type base forte qui donnent accès à des polymères de DPn supérieur à 200. Ces derniers étant précisément ceux qui s'avèrent être appropriés pour les applications des polyaminoacides, l'initiation de la polymérisation des NCA, par des bases fortes, a donc été la voie de synthèse élue par les hommes de l'art.

Mais l'inconvénient de ce type d'initiateurs de polymérisation est, justement, qu'il favorise si bien la réaction que l'on ne contrôle plus le degré de polymérisation, ni la masse molaire $M_w$. On aboutit ainsi à des viscosités réduites extrêmement élevées pour les polyaminoacides. Cela est, bien entendu, pénalisant au regard des opérations de transformation de ces polymères en produits manufacturés.

Il s'ensuit que l'un des objectifs essentiels de la présente invention est de fournir un procédé de synthèse de polyaminoacides à partir de NCA, en milieu liquide et en présence d'initiateurs de type base forte :

- qui permette d'obtenir des produits finis de masse molaire en poids $M_w$ contrôlable et, supérieure ou égale à 50 000 et, plus préférentiellement encore, à 80 000,
- qui offre la possibilité de contrôle de la viscosité réduite de ces produits finis et, mieux encore, qui permette de cibler à l'avance une viscosité réduite donnée,
- et enfin qui soit de mise en oeuvre simple et économique.

Ainsi, c'est après de nombreuses recherches et expériences que la Demanderesse a eu le mérite de mettre en évidence que, pour atteindre cet objectif parmi d'autres, il convient, contre toute attente, d'effectuer la polymérisation des NCA dans un milieu réactionnel liquide comprenant, de l'eau ou un alcool.

Les résultats ainsi obtenus, quant à la maîtrise de la masse moléculaire des polyaminoacides obtenus, sont extrêmement surprenants. En effet, il était, jusqu'alors, parfaitement admis dans l'état de la technique que des réactifs, tels que l'eau et les alcools, pour autant qu'ils soient présents en quantités significatives, induisent une hydrolyse ou alcoolyse des NCA et, par conséquent, conduisent à des oligomères ou polymères de faible masse moléculaire : $DP_R$ inférieur à 200, e. g (cf KRICHELDORF p 60 et BECKER et al. J. A. C. S., 5 janvier 1953, p 737 à 744).

De surcroît, le brevet GB 996 760 enseigne que la mise en oeuvre de quantités infinitésimales d'eau, $10^{-2}$ % en poids par rapport au solvant, provoque une augmentation de la viscosité réduite du polymère final : 3,5 à 4,32 (exemples 1-2).

EP 0 713 505 B1

Par ailleurs, le brevet GB 1 024 393 décrit un procédé de préparation de polyaminoacides consistant, essentiellement, à faire polymériser des NCA d'acides aminés contenant au moins un carbone asymétrique, en présence d'un composé organique chiral à hydrogène actif et d'un composé organo-métallique. Ce dernier représente, au plus, 10 % molaire par rapport aux NCA et le composé organique chiral correspond à 0,3 % molaire par rapport audit composé organo-métallique. Le composé organique chiral peut être choisi, notamment, parmi des alcools.

**EXPOSE DE L'INVENTION :**

En dépit de cela, la présente invention concerne un procédé de préparation de polyaminoacides avec contrôle de leur poids moléculaire, par polymérisation de N-carboxyanhydrides (NCA) d'au moins un acide aminé, à l'aide d'au moins un initiateur et en milieu liquide, selon la présente revendication 1.

Par ce procédé, on peut maîtriser la viscosité réduite des polymères et, par conséquent, leur masse molaire moyenne en poids ($M_w$).

S'agissant de l'alcool, il est, de préférence, sélectionné parmi les monoalcools et/ou les dialcools, linéaires ou cycliques en $C_{1-24}$, ou parmi les alcools aromatiques et, plus préférentiellement, dans la liste suivante : méthanol, éthanol, propanol, butanol, hexanol, phénols, glycols, ces composés étant mis en oeuvre seuls ou en mélange entre eux.

L'alcool utilisé comme moyen de réglage de la viscosité réduite, et donc de la masse moléculaire du polymère fini, est employé à raison de 10 à 3 000 % molaire, de préférence de 10 à 1 000 % molaire et, plus préférentiellement encore, de 10 à 500 % molaire par rapport aux NCA.

L'eau est, quant à elle, employée à raison de 0,1 à 50 % en poids par rapport au solvant organique, de préférence à raison de 0,5 à 10 % en poids et, plus préférentiellement encore, de 1 à 5 % en poids.

Un avantage subsidiaire, lié à l'utilisation d'eau ou d'alcool, est l'accélération de la vitesse de polymérisation.

Ainsi, il est possible, conformément à l'invention, d'établir, pour un ou plusieurs monomères NCA correspondant à des acides aminés différents, une courbe de variation de viscosité réduite du polymère ou du copolymère, en fonction de la concentration d'eau ou d'alcool du milieu réactionnel. Avantageusement, cette courbe peut être utilisée pour déterminer la quantité d'eau et/ou d'alcool, à introduire dans le milieu réactionnel pour obtenir un polymère ou copolymère de viscosité donnée. Cela permet de mettre en adéquation les caractéristiques physiques de la matière première que constituent les polyaminoacides, avec les diverses opérations de transformation et de mise en forme propres à une application finale déterminée (fil, film, matrice ...).

Par ailleurs, il est à souligner que l'utilisation de coréactifs, comme l'eau ou l'alcool, est synonyme de commodité de mise en oeuvre et d'économie.

Les produits de départ du procédé selon l'invention sont des N-carboxyanhydrides d'aminoacides de configuration L, D ou racémique L + D. Il peut s'agir d'un mélange de différents énantiomères et/ou de racémiques. Il est possible de mettre en oeuvre un seul type de monomères constitué par un NCA d'un aminoacide donné ou bien encore différents monomères constitués par des NCA d'aminoacides distincts, de façon à former un copolymère.

Les NCA d'aminoacides peuvent être préparés par toute technique connue et appropriée, comme par exemple celle décrite par w. H. DALY et al., Tetrahedron Letters, vol. 29, n° 6, p 5859, (1988).

Les précurseurs de ces NCA peuvent être choisis parmi les aminoacides suivants, seuls ou en mélange : à partir de glycine, alanine, phénylalanine, leucine, isoleucine, valine, acide α-aminoisobutyrique, acide aspartique, acide glutamique, acide α-aminoadipique, ornithine, lysine, arginine, cystine, méthionine, thréonine, sérine, tyrosine.

La concentration du monomère NCA dans le milieu réactionnel est comprise entre 1 et 40 % en poids par rapport au solvant organique et, de préférence, entre 2 et 20 % en poids et, plus préférentiellement encore, entre 4 et 15 % en poids.

L'initiateur est choisi parmi les amines et/ou les phosphines tertiaires.

Parmi les agents d'amorçage de polymérisation caractéristiques de l'invention, on peut citer, par exemple, la triéthylamine, la triméthylamine, la tri-N ou isopropylamine, la tributylamine, la tricyclohexylamine, le 4,4',4"-tris-diméthylamino-triphénylméthane, des phosphines tertiaires : triphénylphosphine, triméthylphosphine, diméthylphénylphosphine, tributylphosphine, triisopropylphosphine, tricyclohexylphosphine, diphénylméthylphosphine.

Conformément à une disposition préférée de l'invention, on emploie la triéthylamine à titre d'initiateur.

Sur le plan pondéral, l'initiateur représente une quantité molaire comprise entre 0,001 et 1, de préférence entre 0,005 et 0,1 molaire par rapport au NCA.

Selon l'invention, la polymérisation s'opère en milieu liquide, ce qui, en d'autres termes, signifie qu'elle peut être conduite en solution ou en suspension. Dans le cas d'une polymérisation en solution, le (ou les) solvant(s) organique(s) mis en oeuvre est(sont) choisi(s) parmi les classes chimiques suivantes : éthers cycliques ou linéaires, nitriles, halogénés aliphatiques, aromatiques, aromatiques halogénés, dérivés nitrés, esters, cétones, amides, sulfones, sulfoxydes, ou leurs mélanges.

Pour une polymérisation en suspension, le solvant organique est choisi parmi les alcanes : pentane, hexane, octane, décane par exemple, les fluoroalcanes ou leurs mélanges.

A titre d'hydrocarbures aliphatiques halogénés on peut citer, par exemple, le chloroforme, le dichlorométhane, le 1,2-dichlorométhane, le 1,1-dichloroéthane, le 1,2-dichloroéthylène, le 1,1,1-trichloro-éthane, le 1,1,2-trichloroéthane, le trichloroéthylène, le tétrachloroéthylène, le 1,1,2,2-tétrachloroéthane, le 1,1,1,2-tétrachloroéthane, le pentachloroéthane, le 1,2-dichloropropane, le 2,2-dichloropropane, le 1,3-dichloropropane, le 1,2,3-dichloropropane, le dichloroométhane, le 1-bromo-3-chloropropane, le bromure d'isobutylène, le bromoforme.

Les éthers cycliques peuvent être e. g. le dioxane, le tétrahydrofurane.

L'acétonitrile est un exemple de nitrile, le toluène et le xylène des exemples d'aromatique, le nitrométhane un exemple de dérivé nitré et l'acétate de méthyle un exemple d'ester.

Le diméthylformamide (DMF) et le diméthylsulfoxyde (DMSO) sont, respectivement, des exemples d'amide et de sulfoxyde pouvant être mise en oeuvre. A titre d'exemple de sulfone, on peut citer le sulfolane.

Il convient de noter que le dioxane fait partie des solvants organiques particulièrement préférés.

En pratique, cette réaction de polymérisation se déroule à la pression atmosphérique normale, à l'atmosphère ambiante et avec une température de milieu réactionnel comprise entre - 20° C et + 150° C, de préférence entre + 10 et + 90° C.

Ce procédé donne accès à des polyaminoacides (polymères, copolymères) dont les masses molaires et la viscosité réduite sont contrôlables. Cette maîtrise est atteinte sans que cela ne nuise à la facilité de mise en oeuvre ni à l'économie du procédé.

**POSSIBILITE D'APPLICATION INDUSTRIELLE :**

Synthèse de polyaminoacides de caractéristiques mécaniques et rhéologiques adaptées à des applications en tant que biomatériaux.

L'invention sera mieux comprise et ses avantages et variantes de mise en oeuvre ressortiront bien des exemples qui suivent, d'illustration de la polymérisation de NCA d'aminoacides dérivant du glutamate de méthyle et/ou de la leucine.

Les figures annexées faciliteront la compréhension des exemples.

**BREVE DESCRIPTION DES DESSINS :**

- La **fig. 1** montre la variation de la viscosité réduite du polymère en fonction de l'eau ajoutée en milieu de polymérisation.
- La **fig. 2** représente une variation de la constante de vitesse k de la réaction de polymérisation, en fonction de la quantité d'eau dans le milieu réactionnel, dans la polymérisation selon l'exemple 1.
- La **fig. 3** illustre la décroissance de la viscosité du copolymère en fonction de la quantité d'eau rajoutée au milieu réactionnel.
- La **fig. 4** représente le profil cinétique de disparition des NCA, suivi par infra-rouge, dans la polymérisation selon l'exemple 4.

**MEILLEURES MANIERES DE REALISER L'INVENTION :**

**EXEMPLES**

La viscosité réduite, qui est une méthode classique de caractérisation des polyaminoacides, est mesurée à partir d'une solution dans l'acide trifluoroacétique (TFA) à la concentration C de 0,5 g/l. On utilise un tube Ubbelohde, réf. 510 03/0c, placé dans un bain thermostaté à 25° C. Soit $t_0$ le temps d'écoulement du TFA pur et t le temps d'écoulement de la solution de polymère, la viscosité réduite est exprimée de la façon suivante:

$$\eta_{red} = \frac{(t - t_0)}{(t \times C)}$$

La viscosité réduite est le reflet de la masse molaire en poids $M_w$ des polyaminoacides, comme le montre l'exemple 4 ci-après.

EXEMPLE 1 :

**1.1** PRÉPARATION DU NCA-Glu(OMe) = N-carboxyanhydride de glutamate de méthyle.

Dans un réacteur de 0,5 l, muni d'un réfrigérant, d'une ampoule de coulée et d'une agitation magnétique, on charge 32,5 g de glutamate de méthyle et 250 ml de tétrahydrofurane (THF). On introduit dans l'ampoule de coulée une solution de bis-trichlorométhylcarbonate (25 g) dans 38 ml de THF. Le réacteur est placé dans un bain d'huile à la température de 52° C et mis sous balayage d'azote. Dès que la température de la masse réactionnelle atteint 50° C, l'addition de la solution de triphosgène débute. La durée de la coulée est de 20 min. Le milieu réactionnel est agité pendant 25 min supplémentaires, puis filtré pour éliminer les insolubles. Le filtrat est concentré, puis additionné de 300 ml de cyclohexane et refroidi pendant 15 h à - 28° C. Le précipité blanc est filtré. On obtient 30,7 g de NCA-glutamate de méthyle brut. Le produit est recristallisé dans un mélange dichlorométhane/cyclohexane pour donner 28 g de NCA-glutamate de méthyle pur.

Point de fusion = 96,7° C ; enthalpie de fusion = 138,9 j/g ; teneur en chlorures = 0,16 %.

**1.2** POLYMÉRISATION DU NCA-Glu(OMe) :

Dans un réacteur de 250 ml, préalablement purgé à l'azote, on introduit 5 g de NCA-Glu(OMe) et le dioxane, fraîchement distillé sur sodium (75 ml). Le réacteur de polymérisation est placé dans un bain d'huile maintenu à 40° C et le milieu réactionnel agité à 65 tr/min (la température du milieu réactionnel est de 38° C). Après 10 min, on ajoute 0,029 g de triéthylamine. Après 20 h de réaction, le mélange visqueux est dilué avec 75 ml de dioxane. Le poly-Glu (OMe) est isolé par précipitation dans 2l d'eau déminéralisée à température ambiante et lavé deux fois avec 500 ml d'eau avant d'être séché en présence d'anhydride phosphorique et sous vide de la trompe à eau jusqu'à poids constant. On obtient 3,63 g de polymère -Glu(OMe), soit un rendement de 95 %. La viscosité réduite du copolymère, mesurée dans l'acide trifluoroacétique, est de 3,54 dl/g.

**1.3** INFLUENCE DE L'EAU SUR LA VISCOSITÉ DU POLYGLUTAMATE DE MÉTHYLE:

La quantité d'eau, présente dans le milieu réactionnel, a été étudiée dans le domaine 2 %-10 % d'eau par rapport au dioxane (ratio pondéral). Le tableau 1 résume les caractéristiques des polymères obtenus en ajoutant des quantités croissantes d'eau dans le milieu réactionnel.

TABLEAU 1:

| INFLUENCE DE LA QUANTITÉ D'EAU SUR LES CARACTÉRISTIQUES DU POLY-GLU(OME) | | |
|---|---|---|
| EAU/DIOXANE (% poids) | RENDEMENT (%) | $\eta_{red}$ (dl/g) |
| 2,6 | > 93 | 2,42 |
| 5,1 | > 93 | 1,95 |
| 10,4 | > 93 | 1,48 |

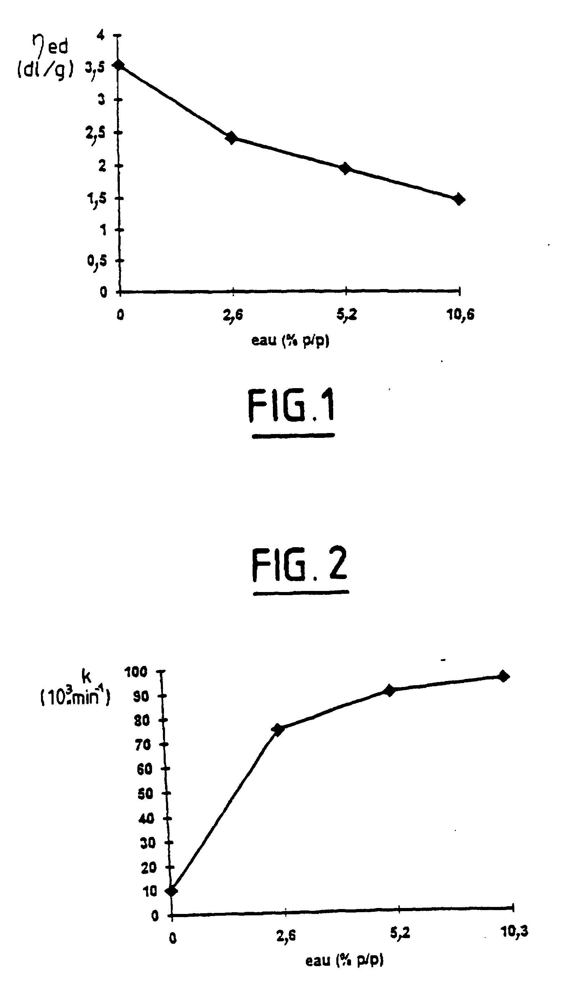

La **fig. 1** annexée montre la variation de la viscosité réduite du polymère en fonction de l'eau rajoutée au milieu de polymérisation.

La **fig. 2** annexée représente la variation de la constante de vitesse k de la réaction de polymérisation, en fonction de la quantité d'eau dans le milieu réactionnel. Elle témoigne de l'effet bénéfique de l'eau sur la cinétique de polymérisation du NCA-Glu(OMe).

**EXEMPLE 2 :**

**2.1** PRÉPARATION DU NCA-Leu.

Dans un réacteur de 0,5 l, muni d'un réfrigérant, d'une ampoule de coulée et d'une agitation magnétique, on charge 26,5 g de leucine et 250 ml de tétrahydrofurane (THF). On introduit dans l'ampoule de coulée une solution de 25 g de bis-trichlorométhylcarbonate (triphosgène) dans 38 ml de THF. Le réacteur est placé dans un bain d'huile à la température de 52° C et mis sous balayage d'azote. Dès que la température de la masse réactionnelle atteint 50° C, l'addition de la solution de triphosgène débute. La durée de la coulée est de 15 min. Le milieu réactionnel est agité pendant 30 min supplémentaires, puis filtré pour éliminer les insolubles. Le filtrat est concentré, additionné de 300 ml de cyclohexane et refroidi pendant 15 h à - 28° C. Le précipité blanc est filtré. On obtient 24,5 g de NCA-leucine brut. Le produit est recristallisé dans un mélange chloroforme/cyclohexane pour donner 20 g de NCA-Leu pur. Point de fusion = 76,9° C ; enthalpie de fusion = 99,8 j/g ; teneur en chlorures = 0,12 %.

**2.2** POLYMÉRISATION DU MÉLANGE NCA-Glu(OMe)/NCA-Leu :

Dans un réacteur de 250 ml, préalablement purgé à l'azote, on introduit, successivement, le mélange NCA-Glu(OMe) et NCA-Leu (5 g à 47 % molaire de NCA-Leu) et le dioxane distillé (75 ml). Le réacteur de polymérisation est placé dans un bain d'huile maintenu à 40° C et le milieu réactionnel agité à 65 tr/min (la température du milieu réactionnel est de 38° C). Après 10 min, la triéthylamine (0,029 g) est ajoutée rapidement. Après 5 h de réaction, le mélange visqueux est dilué avec 75 ml de dioxane et l'agitation augmentée à 150 tr/min, de manière à solubiliser rapidement le polymère. Le copolymère Leu/Glu(OMe) est isolé par précipitation dans 2 l d'eau déminéralisée à température ambiante et lavé deux fois avec 500 ml d'eau, avant d'être séché sous vide en présence d'anhydride phosphorique. On obtient ainsi 3,53 g de copolymère Leu-Glu(OMe), soit un rendement de 95 %. La viscosité réduite du copolymère, mesurée dans l'acide trifluoroacétique, est de 3,7 dl/g.

**2.3** INFLUENCE DE L'EAU SUR LA VISCOSITÉ DU COPOLYMÈRE Leu/Glu(OMe) :

La quantité d'eau, présente dans le milieu réactionnel, a été étudiée entre 0,1 % et 10 % en poids par rapport au dioxane. Le tableau 2 résume les caractéristiques du copolymère en ajoutant des quantités croissantes d'eau dans le milieu réactionnel.

TABLEAU 2 :

| INFLUENCE DE LA QUANTITÉ D'EAU SUR LES CARACTÉRISTIQUES DU COPOLYMÈRE Leu/Glu(OMe) | | | |
|---|---|---|---|
| EAU/DIOXANE (%poids) | RENDEMENT (%) | COMPOSITION (% Leu) | $\eta_{red}$ (dl/g) |
| 0,1 | > 93 | 47 | 3,62 |
| 1 | > 93 | 49 | 3,08 |
| 5 | > 93 | 48 | 1,94 |
| 10 | > 93 | 47 | 1,56 |

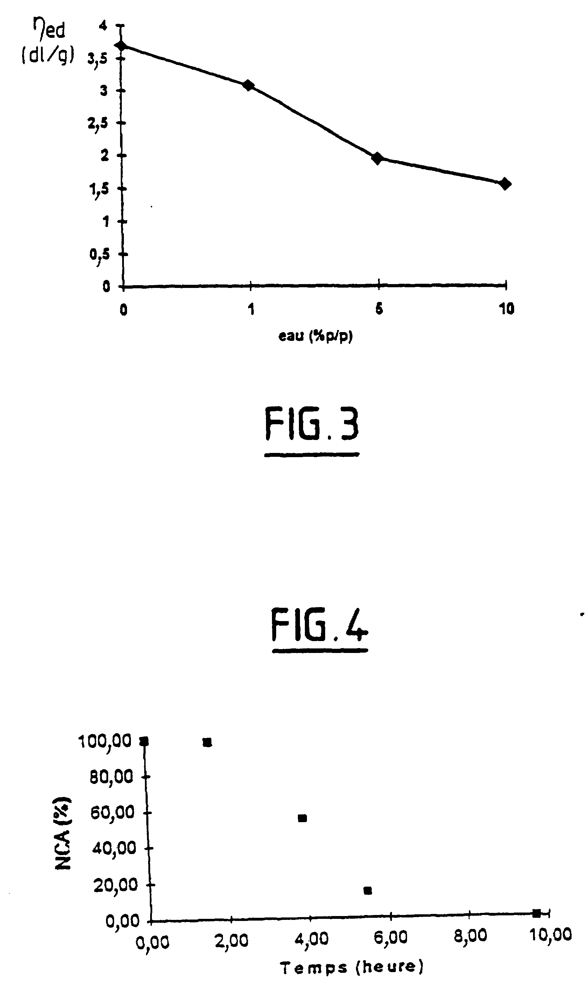

La **fig. 3** annexée illustre la décroissance de la viscosité du copolymère en fonction de la quantité d'eau rajoutée au milieu réactionnel.

**2.4** INFLUENCE DE L'ALCOOL SUR LA VISCOSITE RÉDUITE (MASSE MOLÉCULAIRE) DU POLYMÈRE LEU/GLU (OME) :

L'objectif de cette série de copolymères, composée de quatre produits, est de faire varier la viscosité réduite des copolymères et de maintenir leur composition constante. Les proportions en monomères retenues sont 53 % en GluOMe et 47 % en Leucine.
Le solvant polaire mis en oeuvre est l'éthanol absolu.
La quantité d'éthanol absolu a varié entre 21 et 458 % molaire par rapport aux NCA (soit 0,5 et 8,5 % en poids/dioxane), les proportions en monomères introduites sont restées constantes.
Les résultats sont consignés dans le tableau 3 qui suit.

TABLEAU 3

| EtOH absolu/NCA (% molaire) | EtOH/dioxane (% poids) | Rendement (%) | $\eta_{red}$ (dl/g) |
|---|---|---|---|
| 458 | 8,5 | 92 | 1,75 |
| 275 | 4,9 | 94 | 2,1 |
| 92 | 1,6 | 93 | 2,5 |
| 31 | 0,5 | 92 | 3,1 |

CONDITIONS EXPÉRIMENTALES :

- % molaire Leu/Glu(OMe) = 47/53,
- NCA [Leu + Glu(OMe)] = 20 g (0,1123 moles),
- Dioxanne + EtOH absolu = 300 ml,
- $NEt_3$ = 0,125 g (1 % molaire/NCA),
- T = 40° C,
- agitation = 80 tours/minute.

**EXEMPLE 3 : INFLUENCE DE L'INITIATEUR.**

**3.1** POLYMÉRISATION DU MÉLANGE NCA-Leu/NCA-Glu(OMe) AVEC L'EAU :

L'exemple 2 a été reproduit en omettant la triéthylamine et en ajoutant une quantité d'eau égale à 10 % en poids du dioxane. Le profil cinétique de disparition des NCA, suivi par infra-rouge indique que la polymérisation est terminée en 8 h (cf. **fig. 4** annexée). Le milieu réactionnel est alors traité de la même façon que dans le cas d'une polymérisation classique. On récupère un solide blanc (rendement pondéral = 97 %), dont le spectre RMN $^1$H est identique à celui d'un copolymère Leu-Glu(OMe). La viscosité réduite de ce produit dans l'acide trifluoroacétique est très faible : $\eta_{red}$ = 0,48 dl/g. Cet essai comparatif montre que l'eau, utilisée seule sans initiateur, conduit à la formation d'un copolymère de faible masse molaire.

**EXEMPLE 4 : CORRESPONDANCE MASSE MOLAIRE EN POIDS $M_w$ ET VISCOSITÉ RÉDUITE $\eta_{red}$.**

Trois échantillons (1 à 3) de différents copolymères Leucine/Glutamate de méthyle (Leu/Glu(OMe) ⇔ 47/53 molaire) ont été préparés selon le protocole de l'exemple 2. Leurs masses molaires en poids $M_w$ ont été déterminées par diffusion de la lumière. Leurs viscosités réduites ont, quant à elles, été mesurées comme indiqué ci-avant.
Le tableau 4 de résultats ci-dessous montre clairement que $M_w$ et $\eta_{red}$ varient dans le même sens. Il y a donc une corrélation directe en ces deux grandeurs.

TABLEAU 4

| ECHANTILLONS | $M_w$ | $\eta_{red}$ |
|:---:|:---:|:---:|
| 1 | 325 000 | 1,8 |
| 2 | 290 000 | 1,6 |
| 3 | 100 000 | 1,1 |

**Revendications**

**1.**   Procédé de préparation de polyaminoacides de masse molaire $M_w \geq 50\,000$ , avec contrôle de cette masse molaire $M_w$, par polymérisation de N-carboxyanhydrides (NCA) d'au moins un acide aminé, à l'aide d'au moins un initiateur basique, en milieu liquide et en présence d'un solvant organique,
  **caractérisé en ce qu'**il consiste :
Δ à mettre en oeuvre un initiateur choisi parmi les amines tertiaires et/ou les phosphines tertiaires et à introduire, dans le milieu réactionnel :

- une quantité d'eau comprise entre 0,1 et 50% en poids par rapport au solvant organique ;
- ou une quantité d'alcool comprise entre 10 et 3000 % molaire par rapport aux NCA,
- une quantité de monomères NCA comprise entre 1 et 40 % en poids par rapport à ce solvant organique,
- et une quantité molaire d'initiateur comprise entre 0,001 et 1 par rapport au NCA; Δ et à jouer sur la quantité d'eau ou d'alccol introduite pour contrôler le poids moléculaire des polymères finaux.

**2.**   Procédé selon la revendication 1, **caractérisé en ce que** :

Δ la quantité d'eau introduite est comprise entre 0,5 et 10% en poids, de préférence entre 1 et 5% en poids , par rapport au solvant organique ;

Δ ou la quantité d'alcool introduite correspond à une quantité molaire de 10 à 1000% molaire, par rapport aux NCA

Δ la quantité de monomères NCA est comprise entre 2 et 20% en poids , de préférence entre 4 et 15% en poids , par rapport au solvant organique ;

Δ et la quantité d'initiateur introduite correspond à une quantité molaire de 0,005 à 0,1 par rapport au NCA.

**3.** Procédé selon la revendication 1, **caractérisé en ce que** :

Δ la quantité d'eau introduite est comprise entre 0,5 et 10% en poids, par rapport au solvant organique ;

Δ ou la quantité d'alcool introduite correspond à une quantité molaire de 10 à 500% molaire, par rapport aux NCA.

**4.** Procédé selon l'une quelconque des revendications 1 à 3, **caractérisé en ce qu'**il consiste :

- à établir, pour un (des) polyaminoacide(s) donné(s), la courbe donnant la viscosité réduite des polymères obtenus en fonction de la concentration en eau ou alcool du milieu liquide réactionnel,
- à sélectionner une certaine viscosité réduite our un polyaminoacide visé,
- à déterminer, à l'aide de la courbe, la quantité d'eau ou d'alcool correspondante,
- et à mettre en oeuvre la polymérisation avec cette quantité, de manière à obtenir le polyaminoacide visé.

**5.** Procédé selon l'une quelconque des revendications 1 à 4, **caractérisé en ce que** l'alcool est sélectionné parmi les monoalcools et/ou des dialcools, linéaires ou cycliques en $C_{1-24}$ ou parmi les alcools aromatiques.

**6.** Procédé selon la revendication 5, **caractérisé en ce que** l'alcool est sélectionné dans une liste suivante : méthanol, éthanol, propanol, butanol, hexanol, phénols, glycols, ces composés étant mis en oeuvre seuls ou en mélange entre eux.

**7.** Procédé selon l'une quelconque des revendications 1 à 6, **caractérisé en ce que** le(les) NCA est(sont) obtenu (s)) à partir d'au moins l'un des acides aminés suivants : glycine, alanine, phénylalanine, leucine, isoleucine, valine, acide α-aminoisobutylique, acide pentaméthylène spiroaminoacétique, acide aspartique, acide glutamique, acide α-aminoadipique, ornithine, lysine, arginine, lysine, arginine, cystine, méthionine, thréonine, sérine, tyrosine.

**8.** Procédé selon l'une quelconque des revendications 1 à 7, **caractérisé en ce que** l'on sélectionne le solvant organique parmi : les éthers cycliques ou linéaires, les nitriles, les aliphatiques halogénés, les aromatiques, les aromatiques halogénés, les dérivés nitrés, les esters, les cétones, les amides, les sulfones, les sulfoxydes ou leurs mélanges.

**9.** Utilisation d'eau ou d'alcool pour le contrôle du poids moléculaire de polyaminoacides préparés par polymérisation de N-carboxyanhydrides (NCA) selon le procédé de la revendication 1.


**Patentansprüche**

**1.** Verfahren zur Herstellung von Polyaminosäuren mit der Molmasse $M_w \geq 50\,000$, wobei diese Molmasse $M_w$ gesteuert wird durch Polymerisation von N-Carboxyaminosäureanhydriden (NCA) mindestens einer Aminosäure mit Hilfe von mindestens einem basischen Katalysator in einem flüssigen Medium und unter Beigabe eines organischen Lösungsmittels,

**dadurch gekennzeichnet, dass** es darin besteht,

• einen Katalysator einzusetzen, der unter den tertiären Aminen und/oder den tertiären Phosphinen gewählt wurde, und dem Reaktionsmedium hinzuzufügen:

- eine Quantität Wasser, die zwischen 0,1 und 50 % Gewichtsanteile im Verhältnis zu dem organischen Lösungsmittel beträgt,
- oder eine Quantität Alkohol, die zwischen 10 und 3000 % Molanteile im Verhältnis zu den NCA beträgt,
- eine Quantität NCA-Monomere, die zwischen 1 und 40 % Gewichtsanteile im Verhältnis zu diesem organischen Lösungsmittel beträgt,

-   und eine Molmenge Katalysator, die zwischen 0,001 und 1 im Verhältnis zu den NCA beträgt,

•   und die zugeführte Quantität Wasser oder Alkohol zu variieren, um das Molekulargewicht der sich ergebenden Polymere zu steuern.

2.  Verfahren nach Anspruch 1, **dadurch gekennzeichnet, dass** :

•   die zugeführte Quantität Wasser zwischen 0,5 und 10 % Gewichtsanteile, vorzugsweise zwischen 1 und 5 % Gewichtsanteile im Verhältnis zu dem organischen Lösungsmittel beträgt,
•   die zugeführte Quantität Alkohol einer Molmenge von 10 bis 1000 % im Verhältnis zu den NCA entspricht,
•   die Quantität NCA-Monomere zwischen 2 und 20 % Gewichtsanteile, vorzugsweise zwischen 4 und 15 % Gewichtsanteile im Verhältnis zu dem organischen Lösungsmittel beträgt,
•   und die zugeführte Quantität Katalysator einer Molmenge von 0,005 bis 0,1 im Verhältnis zu den NCA entspricht.

3.  Verfahren nach Anspruch 1, **dadurch gekennzeichnet, dass** :

•   die zugeführte Quantität Wasser zwischen *0,5* und 10 % Gewichtsanteile im Verhältnis zu dem organischen Lösungsmittel beträgt,
•   die zugeführte Quantität Alkohol einer Molmenge von 10 bis 500 % im Verhältnis zu den NCA entspricht.

4.  Verfahren nach einem der Ansprüche 1 bis 3, **dadurch gekennzeichnet, dass** es darin besteht,

-   für eine (die) gegebene(n) Polyaminosäure(n) die Kurve zu bestimmen, die die verringerte Viskosität der hergestellten Polymere in Abhängigkeit von der Konzentration an Wasser oder Alkohol in dem flüssigen Reaktionsmedium angibt,
-   für eine gewünschte Polyaminosäure eine bestimmte verringerte Viskosität zu wählen,
-   mit Hilfe der Kurve die entsprechende Quantität Wasser oder Alkohol zu bestimmen,
-   und die Polymerisation mit dieser Quantität durchzuführen, so dass die gewünschte Polyaminosäure hergestellt wird.

5.  Verfahren nach einem der Ansprüche 1 bis 4, **dadurch gekennzeichnet, dass** der Alkohol unter den einwertigen Alkoholen und/oder den zweiwertigen, linearen oder mit $C_{1-24}$ zyklischen Alkoholen oder unter den aromatischen Alkoholen gewählt wird.

6.  Verfahren nach Anspruch 5, **dadurch gekennzeichnet, dass** der Alkohol aus der folgenden Liste gewählt wird: Methanol, Ethanol, Propylalkohol, Butanol, Hexanol, Phenol, Glykol, wobei diese Komponenten einzeln oder miteinander gemischt verwendet werden können.

7.  Verfahren nach einem der Ansprüche 1 bis 6, **dadurch gekennzeichnet, dass** das (die) NCA aus mindestens einer der folgenden Aminosäuren hergestellt wird (werden): Aminoessigsäure, Alanin, Phenylalanin, Leucin, Isoleucin, Valin, a-Aminoisobuttersäure, spiroaminosaure Pentamethylensäure, Asparginsäure, Glutaminsäure, a-Aminoadipinsäure, Ornithin, Lysin, Arginin, Cystin, Methionin, Threonin, Serin, Tyrosin.

8.  Verfahren nach einem der Ansprüche 1 bis 7, **dadurch gekennzeichnet, dass** das organische Lösungsmittel unter den zyklischen oder linearen Ethern, den Nitrilen, den halogenhaltigen Aliphaten, den Aromastoffen, den halogenhaltigen Aromastoffen, den Nitroderivaten, den Estern, den Ketonen, den Amiden, den Sulfonen, den Sulfoxiden oder deren Mischungen gewählt wird.

9.  Verwendung von Wasser oder Alkohol zur Steuerung des Molekulargewichts von Polyaminosäuren zur Polymerisation von N-Carboxyaminosäureanhydriden (NCA) gemäß dem Verfahren von Anspruch 1.

**Claims**

1.  Method for the preparation of polyaminoacids, which molecular weight $M_w \geq 50\,000$, with control of this molecular weight $M_w$ by polymerization of N-carboxyanhydrides (NCAs) of at least one amino acid, using at least one alkaline initiator in liquid medium, in the presence of an organic solvent,

**characterized in that** it consists essentially in :

- using an initiator chosen from tertiary phosphines and/or amines and introducing into the polymerization medium :

  - an amount of water of between 0.1 and 50% by weight relative to the organic solvent;
  - or an amount of alcohol of between 10 and 3000 mol % relative to the NCAs;
  - an amount of NCA monomers of between 1 and 40 % by weight relative to that organic solvent;
  - an molar amount of initiator of between 0.001 and 1 relative to the NCAs;

- and varying the introduced amount of water or alcohol, in order to control the molecular weight of the final polymers.

2. A method according to claim 1 **characterized in that**:

- the introduced amount of water relative to the organic solvent is between 0.5 and 10% by weight, preferably between 1 and 5% by weight;
- or the introduced amount of alcohol corresponds to a molar amount of between 10 and 1000 mol %, relative to the NCAs ;
- the NCA monomers amount is between 2 and 20 % by weight, preferably between 4 and 15 % by weight, relative to the organic solvent;
- and the introduced amount of initiator corresponds to a molar amount of 0.005 to 0.1, relative to the NCAs.

3. A method according to claim 1, **characterized in that**:

- the introduced amount of water relative to the organic solvent is between 0.5 and 10% by weight ;
- or the introduced amount of alcohol corresponds to a molar amount of between 10 and 500 mol %, relative to the NCAs.

4. A method according to any of the claims 1 to 3, **characterized in that** it consists in:

  - establishing, for one or more given polyaminoacids, a curve showing reduced viscosity of the polymers obtained as a function of the concentration of water or of alcohol in the liquid reaction medium,
  - selecting a certain reduced viscosity for a target polyamino acid,
  - determining, using the curve, the corresponding amount of water or alcohol,
  - and carrying out the polymerization with this amount, so as to obtain the target polyamino acid.

5. A method according to any of the claims 1 to 4, **characterized in that** the alcohol is a linear or cyclic $C_{1-24}$ monoalcohol, dialcohol, a mixture thereof, or an aromatic alcohol.

6. A method according to claim 5, **characterized in that** the alcohol is selected from the group consisting of methanol, ethanol, propanol, butanol, hexanol, phenols, glycols, and mixtures thereof.

7. A method according to any of the claims 1 to 6, **characterized in that** the NCAs are obtained from at least one amino acid selected from the group consisting of glycine, alanine, phenylalanine, leucine, isoleucine, valine, .alpha.-aminoisobutylic acid, pentamethylenespiroaminoacetic acid, aspartic acid, glutamic acid, .alpha.-aminoadipic acid, ornithine, lysine, arginine, cysteine, methionine, threonine, serine, and tyrosine.

8. A method according to any of the claims 1 to 7, **characterized in that** the organic solvent is selected from the group consisting of: cyclic or linear ethers, nitriles, halogenated aliphatics, aromatics, halogenated aromatics, nitro derivatives, esters, ketones, amides, sulfones, sulfoxides, and mixtures thereof.

9. Use of water or alcohol for controlling the molecular weight of polyaminoacids prepared by polymerization of N-carboxyanhydrides (NCAs) according to the method of claim 1.

$\eta$ed
(dl/g)

eau (% p/p)

## FIG.1

## FIG. 2

k
($10^3$.min$^{-1}$)

eau (% p/p)

$\eta_{ed}$ (dl/g)

eau (%p/p)

# FIG.3

# FIG.4

NCA (%)

Temps (heure)